# EUROPÄISCHE PATENTSCHRIFT

(11) **EP 1 440 695 B1**
(45) Veröffentlichungstag und Bekanntmachung des Hinweises auf die Patenterteilung: **01.07.2009**
(21) Anmeldenummer: 04001182.7
(22) Anmeldetag: 21.01.2004
(51) Int. Cl.: A61L 9/015, A61L 9/22, F24F 3/16

(54) **Vorrichtung zur Aktivierung von Luftsauerstoff zum Abbau von VOC-Lasten und Keimen in geschlossenen Räumen sowie zur Aufrechterhaltung einer naturadäquaten Sauerstoffionen-und Ozonkonzentration**
Device for activating oxygen from air for the destruction of VOCs and germs in closed rooms and for maintaining oxygen and ozone concentrations equal to those occuring in nature
Dispositif pour activer l'oxygène provenant de l'air pour la destruction de COVs et des germes ainsi que pour maintenir des concentrations en oxygène et en ozone égales à celles rencontrées dans la nature

(30) Priorität: 21.01.2003 DE 10302397
(43) Veröffentlichungstag der Anmeldung: 28.07.2004
(73) Patentinhaber: Siegmund, Helmut, Dipl.-Ing., 53604 Bad Honnef (DE); Laskowski, Rainer, Dipl.-Ing., 32130 Enger (DE)
(72) Erfinder: Siegmund, Helmut, Dipl.-Ing., 53604 Bad Honnef (DE); Laskowski, Rainer, Dipl.-Ing., 32130 Enger (DE)
(74) Vertreter: Schaumburg, Thoenes, Thurn, Landskron, Eckert

(56) Entgegenhaltungen:
- DE-A- 2 920 813
- US-A- 5 667 563
- US-A- 5 681 533
- US-A1- 2002 014 401

## Beschreibung

Die Erfindung betrifft eine Vorrichtung zum Reduzierung von Keimen sowie VOC-Lasten in der Raumluft, auf Flächen im Raum und den an den Raum angeschlossenen Belüftungsanlagen sowie zur Aufrechterhaltung eines naturadäquaten Verhältnisses von Sauerstoffionen und OZON im Raum über die Luftzuführung oder zentrale Lüftungsanlagen oder über Luftumwälzung durch separate, im Raum platzierte, kombinierte Ionen- und Ozonerzeugungsgeräte sowie deren Regelung in Abhängigkeit von den gemessenen quantitativen VOC-Lasten, den qualitativen VOC-Lasten, den quantitativen Keimbelastungen oder den qualitativen Keimbelastungen.

Bislang wurden Anlagen zum Abbau von Lasten in der Raumluft vorrangig auf Basis der Bildung von Sauerstoffionen PS DE 43 34 956 eingesetzt, wobei der dabei ungewollt gebildete Anteil von Ozon als Störgröße betrachtet und entsprechende Gegenmaßnahmen zum Abbau des Ozons eingeleitet wurden OS DE 100 07 523. Derartige Anlagen werden in ihrer Leistung, d.h. Bildung von Sauerstoffionen, in Abhängigkeit von den im Raum vorhandenen VOC-Lasten gesteuert, wobei keine Beschränkung der Ionenanzahl als primäre Größe vorgenommen wird. Wiederum andere Systeme nutzen gezielt die Bildung von Ozon zum Abbau von VOC- und Keimlasten. Dabei wird wie in OS DE 101 18 078 beschrieben, über eine dielektrisch behinderte Entladung eine bestimmte Menge Ozon oder Sauerstoffionen über einen inneren Regelkreis der Ozonerzeigungseinheit in Abhängigkeit des Volumenstromes erzeugt. Diese Systeme lassen sich in ihrer Ozonerzeugungsleistung über VOC-Sensoren als zusätzliche Regelgröße automatisch einstellen. Wiederum andere Verfahren nutzen die Bildung einer bestimmten Menge Ozon als Oxidationsmittel, um in nachgeschalteten Sorbtionskatalysatoren VOC- und Keimlasten abzubauen. Vorrangig wird bei den Katalysatoren Aktivkohle aufgrund der hohen Aufnahmefähigkeit von VOC und Keimlasten und der Fähigkeit, begrenzte Mengen an Ozon abzubauen, verwendet. Zur Verbesserung des Ozon-Abbaus können dabei zusätzliche Katalysatoren nachgeschaltet werden. Derartige Systeme haben somit die Funktion eines punktuellen Filters, sichern eine VOC- und keimlastreduzierte Zuluft, können aber keinen Einfluß auf die Lastquellen am Entstehungsort im Raum und deren Reduzierung vor Ort nehmen. Alle Systeme ignorieren, dass in der den Menschen umgebenden Aussenluft ein bestimmtes Verhältnis von Ozon zu Sauerstoffionen vorliegt. Dieses Verhältnis besteht in der freien Natur aus 20 - 30 ppb (40-60 µg cm³) Ozon und 100 - 15.000 Kleinionen pro cm³.

Aufgabe der Erfindung ist es, ein Verfahren und eine Anordnung von Systembauteilen zu schaffen, welche einerseits die natürlichen Bedingungen der Außenluft in Bezug auf das Verhältnis von Ozon zu Sauerstoffionen in einem abgeschlossenen Raum sichert und andererseits, durch die vorab erfassten und definierten Außenluftverhältnisse , in einem bestimmten Bereich des Verhältnisses der Sauerstoffionenkonzentration (Kleinionenkonzentration) zu Ozon, VOC-lastabhängig und keimlastabhängig die Menge an erzeugten Sauerstoffionen und Ozon zur gezielten Oxydation der besagten Laststoffe regelt.
Die Anzahl an Raumluftsauerstoffionen (-kleinionen) bezieht sich dabei in ihren Grenzwerten auf insbesondere mind. 100 pro cm³ und maximal 10.000 pro cm³, was einer Situation im Gebirge entspricht. Als oberer Grenzwert für den Ozon-Anteil im Raum wird ein Wert von 30 ppb (60 µg/cm³) definiert, welcher unterhalb der Geruchsschwelle von 40 ppb (80 µg) und im Bereich der natürlichen und als Bio-Ozon bekannten Konzentration der Aussenluft von durchschnittlich 20-30 ppb liegt.

Erfindungsgemäß wird die Aufgabe durch Anspruch 1 gelöst. Die US 5681533 und die US 5667563 beschreiben jeweils ein Gerät, das zur Reinigung und Deodosierung von Luft dient und sowohl einen Ozongenerator als auch eine Ionisierungsvorrichtung enthält.

Dabei werden zusätzlich thermodynamische Daten wie die Temperatur und absolute oder relative Luftfeuchte aufgenommen.

Die Messdatenerfassung der in der Luft enthaltenen Fremdstoffe wie VOC-Lasten und/oder der Keimbelastung erfolgt über Sensoren mit elektrischem digitalen oder analogen Ausgangsignal wie beispielsweise Mischgassensoren oder optoelektronische, mikromechanische Sensoren, Systeme der Nanotechnologie, spektroskopische Sensortechnik, Chemo- oder Biosensoren. Eine Kombination aus verschiedenen Sensoren ist möglich. Die Datenübertragung zur Regelung erfolgt dabei über Datenleitungen oder LON oder kabellos.

Die einzelnen Daten in Form von Quantitäten und/oder Qualitäten können an der elektronischen Steuer- oder Regeleinheit abgelesen werden und stehen über eine Datenschnittstelle als Parallel- oder serielle oder USB-Schnittstelle für Computer oder über Modem/Funkmodem wie z.B. UMTS-Modem oder einer Datenfernleitung oder über LON-Bus einer zusätzlichen externen Ver- und Bearbeitung, Diagnose und Kontrolle, oder Eingriff in die Steuer- oder Regeleinheit zur Verfügung. Eine Kombination aus unterschiedlichen Datenschnittstellen ist möglich.

Die Leistungsteile oder das Leistungsteil wird dabei kraft- oder formschlüssig so in den Kanal oder das Lüftungsgerät eingebracht, dass es mit dem zu behandelnden Luftstrom direkt Kontakt hat und dadurch das gewünschte Verhältnis Sauerstoffionen zu Ozon direkt gebildet werden kann. Eine andere Möglichkeit ist durch Beimengung eines sekundär zugeführten zweiten Luftstromes in welchem durch das Leistungsteil Ozon und Sauerstoffionen gebildet werden. Die Leistung ist dabei so zu wählen, dass nach Mischung beider Luftströme ein Verhältnis von Sauerstoffionen (Kleinionen) zu Ozon adäquat der Außenluft im Verhältnis von insbesondere 100 - 10.000 Ionen pro cm³ und 0 - 30 ppb Ozon erzeugt werden kann.
Die Behandlung größerer Luftmengen wird durch ein entsprechend dimensioniertes Leistungsteil oder durch Kombination aus mehreren kleineren Leistungsteilen, welche durch eine Regler oder eine Steuerung überlagert sind und einzeln angesteuert werden, durchgeführt.

Durch Verwendung von entsprechendem katalytischem Material als Filterelemente, wie z.B. Aktivkohle, können Lastspitzen ausgangsseitig der Luftbehandlungsstrecke zwischengespeichert und kontinuierlich aboxidiert sowie Ozon-Spitzen abgebaut werden.

Die Regelung kann durch einfaches Umschalten als Steuerung oder Messeinrichtung genutzt werden, wobei manuell das Ausgangssignal eingestellt werden kann.
Die Messung von Sauerstoffionen (Kleinionen) und Ozon wird über entsprechende Sensoren im Raum oder ausgangsseitig der Luftbehandlungseinheit oder bei Umluftnutzung eingangsseitig der Luftbehandlungseinheit vorgenommen.

Durch dieses Verfahren und die Schaltungsanordnung wird sichergestellt, dass sich die Raumluft hinsichtlich der Menge an Sauerstoffionen (Kleinionen) und Ozon kontinuierlich in einem Zustand befindet, welcher den natürlichen Lebensbedingungen des Menschen in der mit VOC und Keimen gering belasteten natürlichen Außenluft, nahe kommt und keine Überlastungen der Raumluft durch die einseitige Behandlung von Störgrößen wie z.B. nur der VOC-Last als Reglereingangsgröße, mit Ozon oder Ionen auftreten. Mit diesem Verfahren werden gesundheitliche Gefahren wie z.B. Reizungen oder störende Geruchsempfindungen auf übermäßige Ionenkonzentrationen vermieden.

Vorteilhafte Weiterbildungen der erfindungsgemäßen Anordnung sind den Unteransprüchen zu entnehmen.

Die Erfindung wird durch nachfolgende Prinzipskizzen, Fig. 1 und 2 und Ausführungsbeispiele Fig. 3 bis 5 näher erläutert.

Es zeigen:

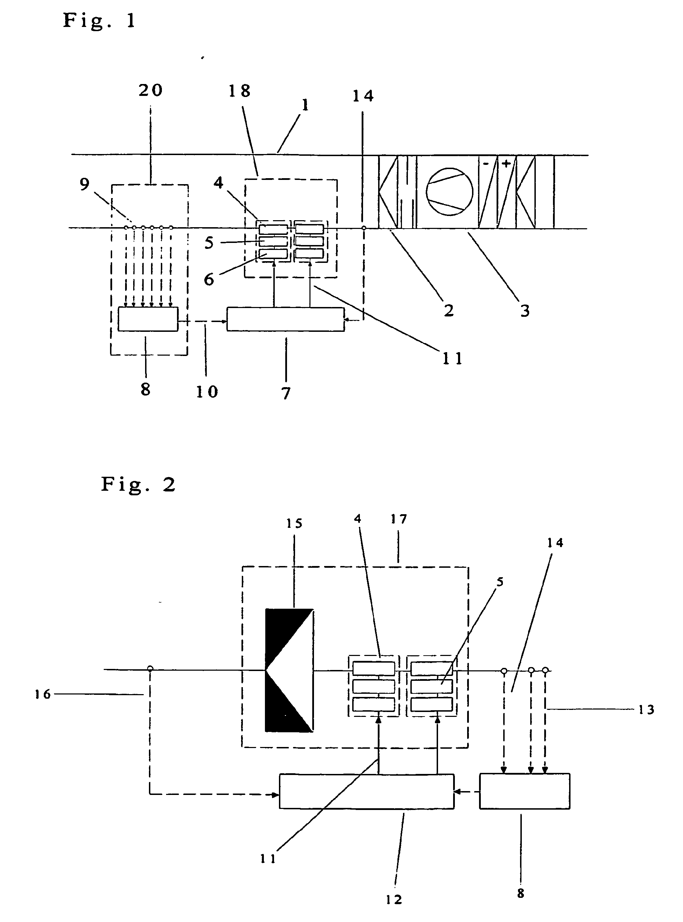

- Fig. 1: Eine nicht erfindungsgemäße Vorrichtung als Grundeinheit
- Fig. 2: eine nicht erfindungsgemäße Vorrichtung als Filtereinheit

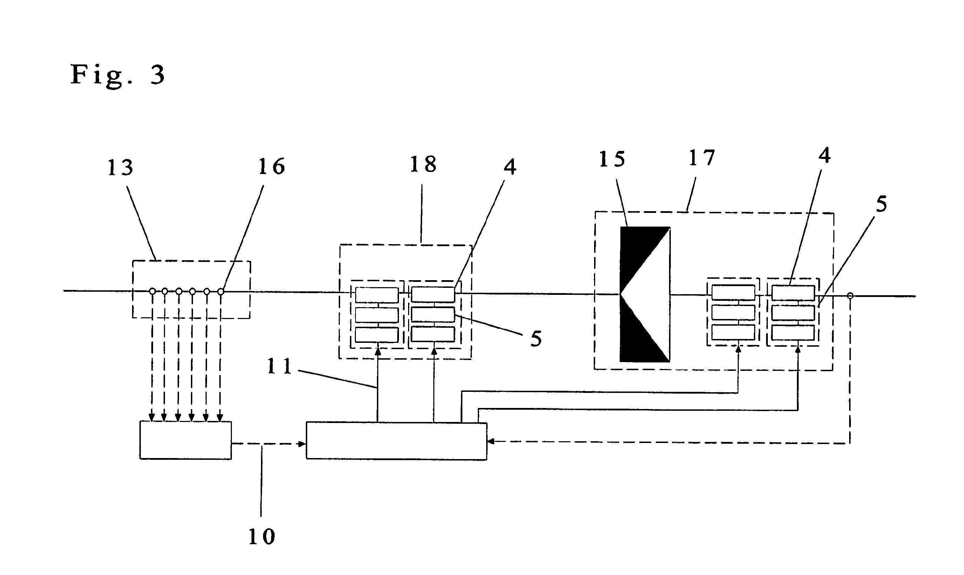

- Fig. 3: Die erfindungsgemäße Vorrichtung zur Vor- und Nachbehandlung von Zuluft

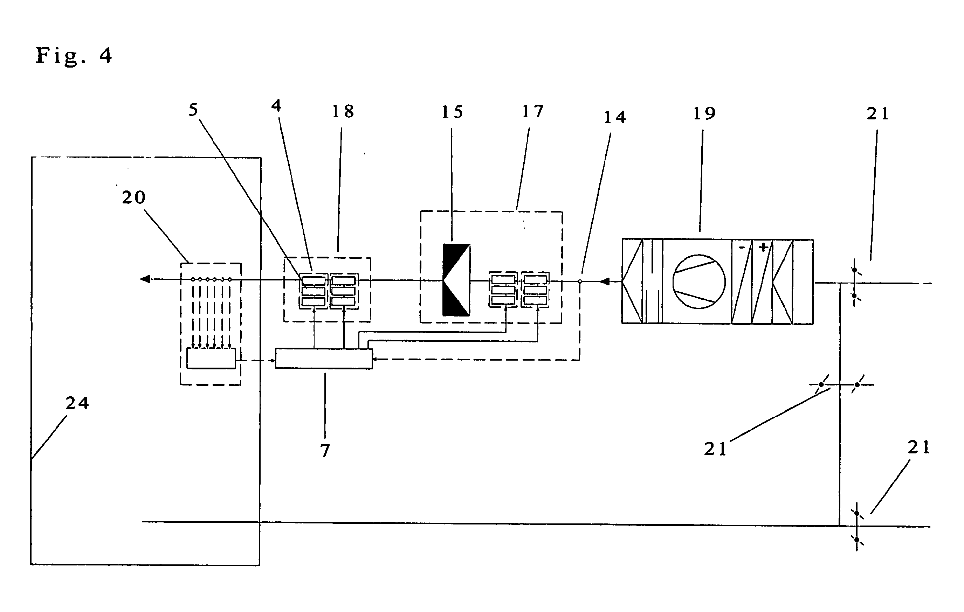

- Fig. 4: Die erfindungsgemäße Vorrichtung als Ausführungsbeispiel in einer zentralen Lüftungsanlage

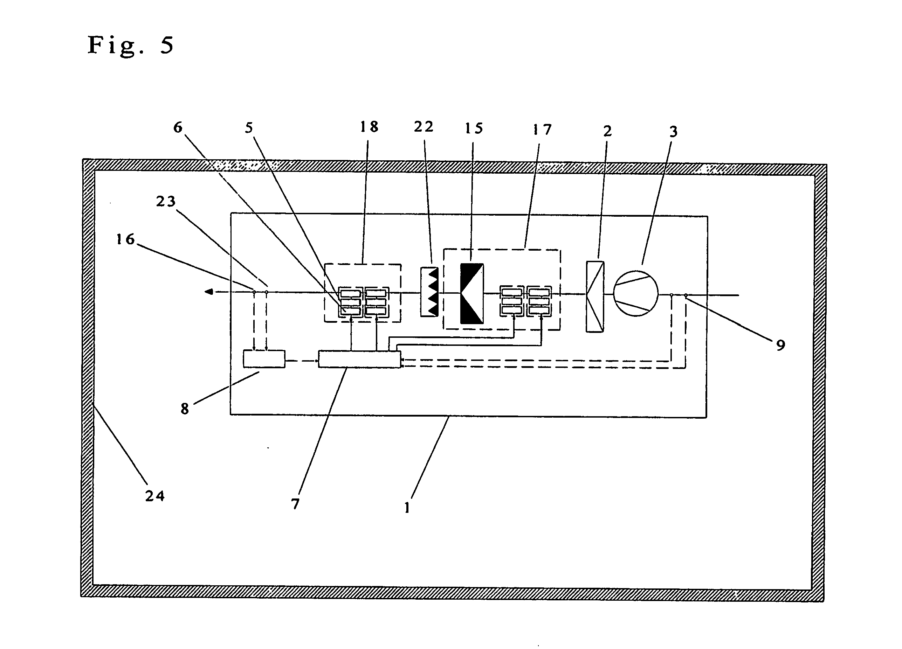

- Fig. 5: Die erfindungsgemäße Vorrichtung als Einbau in ein separates Umluftgerät

In **Fig. 1** ist das grundlegende Verfahren und die Anordnung der Elemente sowie die Regelung dargestellt. In einem Kanal, in welchem Luft zwangsgeführt und transportiert wird (1) befindet sich nach den lufttechnischen Elementen Filter (2) und Ventilator (3) als letztes Einbauteil die oder das Ozonisierungs- und Ionisationsmodul (4) in Kombination mit Leistungsteil (5) und Steuereinheit (6) Das Leistungsteil mit Steuereinheit ist von einem Regler (7) mit vorgeschalteten Messwandler (8) überlagert.
Dabei werden die Störgrößen, wie Fremdbelastung der Luft, nach der Regeleinheit und Regelstrecke mittels geeigneter Mess- und Sensortechnik kontinuierlich aufgenommen (9). Bei den Störgrößen handelt es sich um thermodynamische Größen wie Temperatur und Luftfeuchte, um aerodynamische Größen wie Luftgeschwindigkeit oder Volumenstrom, Menge an Sauerstoffionen und Ozon, der CO₂-Belastung sowie der Fremdbelastung durch luftgetragene Laststoffe wie flüchtige Kohlenwasserstoffe und/oder Keime.
Die ermittelten Daten (10) werden der Regeleinheit (7) zugeführt und über einen vorab in der Regeleinheit programmierten oder digital fest verschalteten Programmablauf ausgewertet, und eine entsprechende Führungsgröße oder Stellgröße für die Leistungsteile (11)ausgegeben.
Für die Datenerfassung der VOC-Lasten werden ein oder mehrere Sensoren verwendet, die getrennt Qualität, d.h. Art der Zusammensetzung und/oder Quantität, d.h. Menge aufnehmen. Die Erfassung erfolgt kontinuierlich unter Abgabe eines elektronisch verwertbaren analogen oder digitalen Signals. In gleicher Form werden Keime qualitativ und quantitativ erfasst und ein entsprechendes Signal der Regeleinheit zur Verfügung gestellt. Als Sensoren können hier optoelektronische Elemente, herkömmliche hochintegrierte mikroelektronische aber auch mikromechanische Sensoren, Systeme der Nanotechnologie, spektroskopische, chemische sowie Biosensoren zum Einsatz kommen.

Die Sensortechnik kann in der Regelung jeweils dem neuesten Stand der Technik angepasst werden.
Als Reglerausgangssignal wird über das der Regelung (7) interne Programm kontinuierlich ein Signal oder eine Signalfolge in Form einer Stellgröße oder Führungsgröße (11) abgegeben, durch welche das Leistungsteil (5) und in Folge die Ionisationseinheit (4) oder Ozoneinheit entsprechend den gemessenen Lasten reagieren kann. Der Programmablauf in der Regelung ist aus Sicherheitsgründen so ausgelegt, dass ohne manuellen Eingriff maximal 30 ppb Ozon und 10.000 Sauerstoffionen/cm³ (Kleinionen/cm³) erzeugt werden können. Dabei ermöglicht der Programmablauf in der Regelung die gezielte Ionisation von Sauerstoff als Oxydationsmittel in einem Regelbereich von 0 - 10.000 Sauerstoffionen pro cm³ (Kleinionen/cm³), und die Bildung von Ozon in einem Bereich von 0 - 30 ppb.
Durch manuelles Aufheben einer sicherungstechnischen Schaltung ist die Erzeugung von höheren Konzentrationen an Sauerstoffionen (Kleinionen) und Ozon und die freie Programmierung der Regelung möglich, wobei beim Betrieb unter diesen Leistungsstufen entsprechende dauernde markante Warnsignale abgegeben werden können.

**Fig. 2** zeigt eine Ausführung eines nicht erfindungsgemäßen Verfahrens und einer solchen Systembaugruppenanordnung. Dabei wird die Systemanordnung als Filtereinheit mit einem Aktivkohlefilter als Sorbtionskatalysator genutzt. Eine Steuereinheit (12), bei welcher die Störgrößen (13) wie Fremdbelastung der Luft mit VOC und Keimen qualitativ und quantitativ vor der Steuereinheit und dem Leistungsteil (5) mittels geeigneter Mess- und Sensortechnik kontinuierlich aufgenommen werden, liefert eine entsprechende Stellgröße (11) für das Leistungsteil (5) nach einem vorab in der Steuereinheit programmierten oder digital fest verschalteten Programmablauf. Als weiter zugeführte Störgröße wird die Luftgeschwindigkeit (14) aufgenommen, aus welcher im Programm der Steuereinheit der zu behandelnde Volumenstrom ermittelt wird. Durch die Aufnahme von Fremdbelastungen vor der im Umluftbetrieb genutzten Steuereinheit oder im Raum wird eine eindeutige Aussage zur Belastung im Raum vorgenommen. Treten größere Lasten an VOC oder Keimen im Raum (24) auf, wird nach dem Programmalgorithmus der Steuerung der Anteil an erzeugtem Ozon erhöht. Die VOC- und Keimlasten werden im Sorbtionskatalysator (15) zwischengespeichert und mittels des Ozons und die daraufhin erfolgte Bildung von -OH-Radikalen und Wasserstoffsuperoxiden aus Wasser, welches über die relative Luftfeuchtigkeit im Katalysator zum Teil mit gebunden wird, im Sorbtionskatalysator aboxidiert. Bei steigenden VOC- oder Keimlasten im Raum ist das System so geschalten, dass eine Erhöhung des zu behandelnden Volumenstromes stattfindet.
Treten Ozon-Lasten durch den Sorbtionskatalysator durch, erfolgt über einen nachgeschalteten Ozon-Sensor (16) der der Regelung aufgeschaltet ist, eine Absenkung der Ozon-Erzeugung auf das notwenige und zugelassene Maß.

**Fig. 3** stellt eine weitere bevorzugte Ausführung der erfindungsgemäßen Anordnung dar. Bei dieser Ausführung werden die Systembauteile so angeordnet und logisch miteinander verknüpft, dass einerseits luftgetragene hohe VOC- und Keimlasten in einer Filtereinheit abgebaut werden und zum anderen der dem Raum zugeführte Luftstrom so behandelt wird, dass die gewünschte Raumluftkonzentration an Sauerstoffionen (Klerinionen) und Ozon gebildet und die VOC- sowie Keimlasten am Entstehungsort im Raum zusätzlich abgebaut werden können. Als erste Systembaugruppe wird die Filtereinheit (17) in Richtung Luftvolumenstrom angeordnet. Die luftgetragenen VOC- und Keimlasten werden im Sorbtionskatalysator zwischengespeichert und mittel des Ozons und die daraufhin erfolgte Bildung von -OH-Radikalen und Wasserstoffsuperoxiden aus Wasser, welches über die relative Luftfeuchtigkeit im Katalysator zum Teil mit gebunden wird, im Sorbtionskatalysator aboxidiert. Bei steigenden VOC- oder Keimlasten im Raum ist das System so geschalten, dass eine Erhöhung des zu behandelnden Volumenstromes stattfindet. Treten Ozon-Lasten durch den Sorbtionskatalysator durch, erfolgt über den nachgeschalteten Ozon-Sensor (16) der der Regelung aufgeschaltet ist, eine Absenkung der Ozon-Erzeugung auf das notwenige und zugelassene Maß. Dieser Systembaugruppe nachgeordnet ist ein Behandlungsmodul (18), welches die dem Raum zugeführte Luft auf die entsprechende Konzentration an Sauerstoffionen (Kleinionen) und Ozon bringt. Für die gesamte Systemanordnung werden die Störgrößen, nach dem Behandlungsmodul (18) mittels geeigneter Mess- und Sensortechnik kontinuierlich aufgenommen. Bei den zu messenden Größen handelt es sich um thermodynamische Größen wie Temperatur und Luftfeuchte, um aerodynamische Größen wie Luftgeschwindigkeit oder Volumenstrom, den Ionisations- und Ozonisierungsgrad, CO₂-Belastung sowie Fremdbelastung durch luftgetragene Laststoffe wie flüchtige Kohlenwasserstoffe und/oder Keime .

Die ermittelten Daten (10) werden der Regeleinheit (7) zugeführt und über einen vorab in der Regeleinheit programmierten oder digital fest verschaltetem Programmablauf ausgewertet, entsprechende Stell- oder Führunsggrößen (11) für die Leistungsteile (5) der Filter- und Behandlungseinheit (17,18) ausgegeben.
Für die Datenerfassung der VOC-Lasten werden ein oder mehrere Sensoren verwendet, die getrennt Qualität, d.h. Art der Zusammensetzung und/oder Quantität, d.h. Menge aufnehmen. Die Erfassung erfolgt kontinuierlich unter Abgabe eines elektronisch verwertbaren analogen oder digitalen Signals. In gleicher Form werden Keime qualitativ und quantitativ erfasst und ein entsprechendes Signal der Regeleinheit zur Verfügung gestellt. Als Sensoren können hier optoelektronische Elemente, herkömmliche hochintegrierte mikroelektronische aber auch mikromechanische Sensoren, Systeme der Nanotechnologie oder spektroskopische, chemische sowie Biosensoren zum Einsatz kommen. Die Sensortechnik kann in der Regelung jeweils dem neuesten Stand der Technik angepasst werden.
Als Regelgröße wird über das der Regelung (7) interne Programm kontinuierlich Signale oder eine Signalfolge in Form von Stellgrößen oder Führungsgrößen (11) abgegeben, durch welche die Leistungsteile (5) und in Folge die Ionisationseinheiten und Ozonerzeugungseinheiten (4) entsprechend den gemessenen Lasten reagieren können. Der Programmablauf in der Regelung ist aus Sicherheitsgründen so ausgelegt, dass ohne manuellen Eingriff maximal 30 ppb Ozon und 10.000 Sauerstoffionen/cm³ (Kleinionen/cm³) erzeugt werden können. Dabei ermöglicht der Programmablauf in der Regelung die gezielte Ionisation von Sauerstoff als Oxydationsmittel in einem Regelbereich von 0 - 10.000 Sauerstoffionen pro cm³ (Kleinionen/cm³), aber auch die Bildung von Ozon in einem Bereich von 0 - 30 ppb. Treten größere Lasten an VOC oder Keimen im Raum auf, wird nach dem Programmalgorithmus der Regelung der Anteil an erzeugtem Ozon erhöht.
Durch manuelles Aufheben einer entsprechenden sicherungstechnischen Schaltung ist die Erzeugung von höheren Konzentrationen an Sauerstoffionen (Kleinionen) und Ozon und die freie Programmierung der Regelung möglich, wobei beim Betrieb unter diesen Leistungsstufen entsprechende dauernde markante Warnsignale abgegeben werden können.

### Ausführungsbeispiel 1

### Anwendung in einer zentralen Lüftungsanlge

Fig. 4 stellt das Verfahren eine Systemkonfiguration für eine zentrale Lüftungsanlge mit der Möglichkeit des Umluftbetriebes dar. Dabei werden die Leistungsteile (5) form- oder kraftschlüssig und luftdicht in den Zuluftkanal nach der raumlufttechnischen Anlage (19) eingebaut. In Luftvolumenstromrichtung, nach der zentralen Lüftungsanlage, wird zunächst eine Systemeinheit zur Bildung von Ozon und nachgeordnet ein Sorbtionskatalysator als Filtereinheit (17) geschaltet. Als Sorbtionskatalysator findet eine Legierung aus Metall Verwendung, welche durch eine große Oberfläche Anlagerungsmöglichkeiten für VOC und Keime bietet und auf die Reaktionsgeschwindigkeit von -OH und Wasserradikalen mit organischen Substanzen und Keimen wirkt. Nach diesem Modul werden weiter Ionisationseinheiten und Ozon-Erzeugungseinheiten als Behandlungsmodul (18) plaziert. In Folge wird die Luftgeschwindigkeit im Kanal zur Ermittlung des Luftvolumenstromes aus Luftgeschwindigkeit und Kanalqerschnitt gemessen (14). Im Raum (24) ist an einer expliziten Stelle ein Kombinationssenor aus Ionensensor, Ozon-Sensor und VOC-Sensor sowie Keimsensor (20) angebracht. Alle Messgrößen geben ihre Daten über einen LON-Bus an die allen Leistungsteilen überlagerte Regelung (7) ab, in welcher die Daten nach einem bestimmten Programmalgorithmus verarbeitet werden und die Stellgrößen (11) an die Leistungseinheiten (5) oder die den Leistungseinheiten angeschlossenen internen Regelungen oder Steuerungen (6) abgibt. Dabei werden die Ozon-Produktion vor dem Katalysator (15) sowie die nach dem Katalysator geschalteten Leistungsteile getrennt mit jeweils einer eigenen Stellgröße oder Führungsgröße angesteuert. Ein weiteres Stellsignal wird an die Umluftklappen (21) der Lüftungsanlage geleitet. Die logische Verknüpfung erfolgt so, dass bei Lastaufnahme durch den VOC- oder Keimsensor die Leistung des Ozon-Moduls vor dem Katalysator erhöht wird. Liegen weiterhin hohe Lasten vor, wird in einem zulässigen Bereich bis 10.000 Sauerstoffionen/cm³ (Kleinionen/cm³) und 30 ppb Ozon im Raum die Leistung der nach dem Katalysator geschalteten Ionisationsmodule und Ozon-Erzeuger erhöht. Liegen nach diesem Schritt immer noch hohe VOC- oder Keimlasten im Raum vor, so wird über ein Stellsignal die Umluftklappe angesteuert und ein höherer Außenluftanteil zur zusätzlichen Lastverdünnung in den Raum befördert. Mit dieser Regelung kann ein gleich bleibend, gesundes Raumklima garantiert und eine bedarfsabhängige Regelung der Lüftungsanlage vorgenommen werden.

### Ausführungsbeispiel 2

### Einbau in ein separates Umluftgerät

**Fig. 5** zeigt die Anwendung in einem einfachen Umluftgerät zum Abbau von VOC-Lasten in kleineren Räumen ohne zentrale Lüftungsanlage. Dabei wird in einer Luftzwangsführung (1) nach dem Ventilator (3) und einer Vorfiltereinheit (2) eine Systemeinheit zur Bildung von Ozon und nachgeordnet ein Sorbtionskatalysator aus Aktivkohle mit einem max. Druckverlust von 100 Pa als Filtereinheit (17) geschaltet. Nach dem Sorbtionskatalysator folgt eine Einheit zum Abbau von aus dem Katalysator durchschlagenden Ozon-Belastungen (22) in Form eines Metalllegierungskatalysators mit geringem Druckverlust. In Folge werden Ionisationsmodule und Ozonerzeugungsmodule als Behandlungseinheit (18) so geschalten, dass durch die allen Leistungseinheiten überlagerte Regelung das entsprechende Verhältnis von Sauerstoffionen (Kleinionen) und Ozon dem Raum zugeführt werden kann. Die notwendigen Sensordaten VOC und Luftgeschwindigkeit zur Ermittlung des zu behandelnden Volumenstromes werden eingangsseitig des Gerätes aufgenommen (9), der Ozon-Gehalt und Ionisationsgrad der Luft werden ausgangsseitig aufgenommen (16,23). Alle aufgenommenen Daten werden der Regelung (7) zugeführt, über einen Programmalgorithmus verarbeitet und die notwendigen Stellsignale oder Führungsgrößen einzeln und voneinander unabhängig den Leistungseinheiten (5) oder der den Leistungseinheiten verbundenen inneren Steuerung oder Regelung (6) zugeführt. Die Regelung (7) kann auf manuellen Betrieb umgestellt und als Steuerung betrieben werden.

### Bezugszeichenliste

- 1: Luftzwangsführung
- 2: Vorfilter
- 3: Ventilator
- 4: Ionisations- oder Ozon-Erzeugungsmodul
- 5: Leistungsteil
- 6: Steuereinheit
- 7: Regler
- 8: Messwandler
- 9: Sensoren
- 10: Daten/Datenleitung
- 11: Stell- oder Führungsgröße
- 12: Steuereinheit
- 13: Störgrößen
- 14: Luftgeschwindigkeitssensor
- 15: Sorbtionskatalysator
- 16: Ozon-Sensor
- 17: Filtereinheit
- 18: Behandlungseinheit
- 19: Raumlufttechnische Anlage
- 20: Kombinationssensor
- 21: Umluftklappe
- 22: Katalysator zum Ozon-Abbau
- 23: Ionensensor
- 24: Raum

## Patentansprüche

1. Vorrichtung zur Reduzierung von Keimen und VOC-Lasten in einem Raum (24), zur Aufrechterhaltung eines naturadäquaten Verhältnisses von Sauerstoffionen und Ozon im Raum und zum Zuführen von Zuluft in den Raum mit maximal 10.000 Sauerstoffionen/cm3 und maximal 30ppb Ozon,
welche folgende Merkmale umfasst:
- einen Kanal (1) zur Zwangsführung von Luft in Richtung des Raumes (24),
- im Kanal (1) Strömungsrichtung hintereinander angeordnet
- einen Ventilator (3),
- einen Filter (2),
- eine Filtereinheit (17) mit einer Systemeinheit zur Bildung von Ozon und nachgeordnet einem Sorbtionskatalysator (15) zum Aboxidieren der VOC- und Keimlasten,
- eine Behandlungseinheit (18) umfassend eine lonisationseinheit und eine Ozonerzeugungseinheit (4) mit je einem Leistungsteil (5) und einer Steuereinheit (6),
wobei
- die Steuereinheiten (6) mit einem ihnen übergeordneten Regler (7) verbunden sind, der mit Sensoren (16,20) zum Erfassen der VOC- und Keimbelastung, der Ozonkonzentration und der Ionenkonzentration im Raum (24) oder am Kanalausgang und zum Erfassen der Luftgeschwindigkeit am Eingang der Filtereinheit (17) verbunden ist, wobei
- die Ozon-Produktion vor dem Sorptionskatalysator (15) sowie die nach dem Katalysator geschalteten Leistungsteile durch den Regler (7) getrennt mit jeweils einer eigenen Stellgröße oder Führungsgröße in Abhängigkeit von den durch die Sensoren gemessenen Größen ansteuerbar sind.

2. Vorrichtung zur Reduzierung von Keimen und VOC-Lasten in einem Raum (24), zur Aufrechterhaltung eines naturadäquaten Verhältnisses von Sauerstoffionen und Ozon im Raum und zum Zuführen von Zuluft in den Raum mit maximal 10.000 Sauerstoffionen/cm3 und maximal 30ppb Ozon,
welche folgende Merkmale umfasst:
- einen Kanal (1) zur Umwälzung der Luft in dem Raum (24),
- im Kanal (1) in Strömungsrichtung hintereinander angeordnet
- einen Ventilator (3),
- einen Filter (2),
- eine Filtereinheit (17) mit einer Systemeinheit zur Bildung von Ozon und nachgeordnet einem Sorbtionskatalysator (15) zum Aboxidieren der VOC- und Keimlasten,
- eine Behandlungseinheit (18) umfassend eine lonisationseinheit und eine Ozonerzeugungseinheit (4) mit je einem Leistungsteil (5) und einer Steuereinheit (6),
wobei
- die Steuereinheiten (6) mit einem ihnen übergeordneten Regler (7) verbunden sind, der mit Sensoren (16,20) zum Erfassen der Ozonkonzentration und der Ionenkonzentration am Kanalausgang und zum Erfassen der VOC Belastung und der Luftgeschwindigkeit am Eingang der Filtereinheit (17) verbunden ist, wobei
- die Ozon-Produktion vor dem Sorptionskatalysator (15) sowie die nach dem Katalysator geschalteten Leistungsteile durch den Regler (7) getrennt mit jeweils einer eigenen Stellgröße oder Führungsgröße in Abhängigkeit von den durch die Sensoren gemessenen Größen ansteuerbar sind.

3. Vorrichtung nach Anspruch 1, **dadurch gekennzeichnet, dass** der Kanal (1) Teil einer Lüftungsanlage für den Raum (24) ist, die so ausgeführt ist, dass durch Stellsignale des Reglers (7) in der Lüftungsanlage angeordnete Umluftklappen (21) betätigbar sind, um einen höheren Außenluftanteil zur zusätzlichen Lastverdünnung in den Raum zu befördern.

4. Vorrichtung nach einem der Ansprüche 1 bis 3, **dadurch gekennzeichnet, dass** der Regler (7) auf manuellen Betrieb umstellbar ist.

5. Vorrichtung nach einem der Ansprüche 1 bis 4, **dadurch gekennzeichnet, dass** Sensoren für folgende weitere Störgrößen als Eingangssignale für den Regler (7) verwendbar sind:
Temperatur, absolute oder relative Feuchte, Dichte, Luftdruck, Volumenstrom, Massestrom, Gaszusammensetzung, Art der Gase, Menge der Gase, Ionisationsgrad der Luft.

6. Vorrichtung nach einem der Ansprüche 1 bis 5, **dadurch gekennzeichnet, dass** die Störgrößen optisch über ein Display oder akustisch zum Beispiel durch einen Signalton bei Überschreiten eines Sollwertes darstellbar sind.

7. Vorrichtung nach einem der Ansprüche 1 bis 6, **dadurch gekennzeichnet, dass** die Störgrößen über eine oder mehrere Schnittstellen wie serielle Datenschnittstelle, parallele Datenschnittstelle, USB-Schnittstelle oder Internetmodem, Funkmodem/UMTS oder LON extern abrufbar sind.

8. Vorrichtung nach einem der Ansprüche 1 bis 7, **dadurch gekennzeichnet, dass** die Störgrößen Sensorsignale in dem Regler (7) befristet abspeicherbar und bei Bedarf auf einen Massenspeicher übertragbar oder als Ausdruck abrufbar sind.

9. Vorrichtung nach einem der Ansprüche 1 bis 8, **dadurch gekennzeichnet, dass** die Sensoren von Mikromechanischen Bauteilen, Systemen der Nanotechnologie, optischen, optoelektronischen oder chemischen Sensoren, Biosensoren oder Kombinationen aus diesen gebildet sind.

## Claims

1. A device for reducing germs and VOC loads in a room (24), for maintaining in the room a ratio of oxygen ions to ozone which is adequate to the one occurring in nature and for supplying fresh air into the room with a maximum of 10,000 oxygen ions/cm³ and a maximum of 30ppb ozone,
the device comprising the following features:
- a channel (1) for the constrained supply of air towards the room (24),
- in the channel (1), arranged behind one another in the direction of flow,
- a ventilator (3)
- a filter (2),
- a filter unit (17) having a system unit for the formation of ozone and downstream thereof a sorption catalyst (15) for the elimination by oxidation of the VOC and germ loads,
- a treatment unit (18) comprising a ionization unit and an ozone production unit (4), each having a power module (5) and a control unit (6), wherein
- the control units (6) are connected to a superordinated controller (7) which is connected to sensors (16, 20) for detecting the VOC and germ loads, the ozone concentration and the ion concentration in the room (24) or at the channel exit, and for detecting the air speed at the entrance of the filter unit (17), wherein
- the ozone production upstream of the sorption catalyst (15) as well as the power modules arranged downstream of the catalyst can be activated separately by the controller (7) with respectively one own control variable or reference variable as a function of the variables measured by the sensors.

2. A device for reducing germs and VOC loads in a room (24), for maintaining in the room a ratio of oxygen ions to ozone which is adequate to the one occurring in nature and for supplying fresh air into the room with a maximum of 10,000 oxygen ions/cm³ and a maximum of 30ppb ozone,
the device comprising the following features:
- a channel (1) for the circulation of the air in the room (24),
- in the channel (1), arranged behind one another in the direction of flow,
- a ventilator (3)
- a filter (2),
- a filter unit (17) having a system unit for the formation of ozone and downstream thereof a sorption catalyst (15) for the elimination by oxidation of the VOC and germ loads,
- a treatment unit (18) comprising a ionization unit and an ozone production unit (4), each having a power module (5) and a control unit (6), wherein
- the control units (6) are connected to a superordinated controller (7) which is connected to sensors (16, 20) for detecting the ozone concentration and the ion concentration at the channel exit and for detecting the VOC load and the air speed at the entrance of the filter unit (17), wherein
- the ozone production upstream of the sorption catalyst (15) as well as the power modules arranged downstream of the catalyst can be activated separately by the controller (7) with respectively one own control variable or reference variable as a function of the variables measured by the sensors.

3. The device according to claim 1, **characterized in that** the channel (1) forms part of a ventilating system for the room (24), which ventilating system is designed such that air admission flaps (21) arranged in the ventilating system can be actuated by control signals of the controller (7) in order to carry a higher proportion of external air into the room for an additional dilution of the load.

4. The device according to one of the claims 1 to 3, **characterized in that** the controller (7) can be switched to manual operation.

5. The device according to one of the claims 1 to 4, **characterized in that** the sensors can be used as input signals for the controller (7) for the following further disturbance variables:
temperature, absolute or relative humidity, density, air pressure, volume flow, mass flow, gas composition, type of gases, quantity of gases, degree of ionization of the air.

6. The device according to one of the claims 1 to 5, **characterized in that** the disturbance variables can be represented optically via a display or acoustically, for example, by a signal tone when a desired value is exceeded.

7. The device according to one of the claims 1 to 6, **characterized in that** the disturbance variables can be retrieved via one or more interfaces such as a serial data interface, a parallel data interface, a USB interface or an internet modem, a radio modem/UMTS or LON.

8. The device according to one of the claims 1 to 7, **characterized in that** the disturbance variables can be stored in the controller (7) for a limited amount of time and, if necessary, can be transferred to a mass storage or retrieved in the form of a printout.

9. The device according to one of the claims 1 to 8, **characterized in that** the sensors are formed of micromechanical components, systems of the nanotechnology, optical, optoelectronical or chemical sensors, biosensors, or combinations thereof.

## Revendications

1. Dispositif pour réduire les germes et les charges de COV dans une pièce (24), pour maintenir dans la pièce un rapport d'ions oxygène et d'ozone égal à celui rencontré dans la nature, et pour acheminer de l'air frais dans la pièce avec 10 000 ions d'oxygène/cm³ au maximum et 30 ppb d'ozone au maximum, lequel dispositif comporte les caractéristiques suivantes :
- un conduit (1) pour le guidage forcé de l'air en direction de la pièce (24),
- dans le conduit (1) sont disposés les uns derrière les autres dans le sens de l'écoulement :
-- un ventilateur (3),
-- un filtre (2),
-- une unité de filtration (17) avec une unité système pour la formation de l'ozone et, en aval de celle-ci, un catalyseur à sorption (15) pour éliminer par oxydation les charges de COV et de germes,
-- une unité de traitement (18) comportant une unité d'ionisation et une unité de production d'ozone (4), munies chacune d'un élément de puissance (5) et d'une unité de commande (6), sachant que
- les unités de commande (6) sont reliées à un régulateur (7) qui leur est supérieur et qui est relié à des capteurs (16, 20) destinés à détecter la charge de COV et de germes, la concentration de l'ozone et la concentration des ions dans la pièce (24) ou au niveau de la sortie du conduit, et à détecter la vitesse de l'air au niveau de l'entrée de l'unité de filtration (17),
- la production d'ozone en amont du catalyseur à sorption (15), ainsi que les éléments de puissance montés en aval du catalyseur pouvant être activés de manière séparée par le régulateur (7), avec respectivement une grandeur de réglage ou grandeur de commande propre en fonction des grandeurs mesurées par les capteurs.

2. Dispositif pour réduire les germes et les charges de COV dans une pièce (24), pour maintenir dans la pièce un rapport d'ions oxygène et d'ozone égal à celui rencontré dans la nature, et pour acheminer de l'air frais dans la pièce avec 10 000 ions d'oxygène/cm³ au maximum et 30 ppb d'ozone au maximum, lequel dispositif comporte les caractéristiques suivantes :
- un conduit (1) pour la circulation de l'air dans la pièce (24),
- dans le conduit (1) sont disposés les uns derrière les autres dans le sens de l'écoulement :
-- un ventilateur (3),
-- un filtre (2),
-- une unité de filtration (17) avec une unité système pour la formation de l'ozone et, en aval de celle-ci, un catalyseur à sorption (15) pour éliminer par oxydation les charges de COV et de germes,
-- une unité de traitement (18) comportant une unité d'ionisation et une unité de production d'ozone (4), munies chacune d'un élément de puissance (5) et d'une unité de commande (6), sachant que
- les unités de commande (6) sont reliées à un régulateur (7) qui leur est supérieur et qui est relié à des capteurs (16, 20) destinés à détecter la concentration de l'ozone et la concentration des ions au niveau la sortie du conduit, et à détecter la charge de COV et la vitesse de l'air au niveau de l'entrée de l'unité de filtration (17),
- la production d'ozone en amont du catalyseur à sorption (15), ainsi que les éléments de puissance montées en aval du catalyseur pouvant être activés de manière séparée par le régulateur (7), avec respectivement une grandeur de réglage ou grandeur de commande propre en fonction des grandeurs mesurées par les capteurs.

3. Dispositif selon la revendication 1, **caractérisé en ce que** le conduit (1) fait partie d'une installation de ventilation de la pièce (24), qui est réalisée de telle sorte que des volets de circulation d'air (21), montés dans l'installation de ventilation, peuvent être activés par les signaux de réglage du régulateur (7) pour acheminer dans la pièce (24) une plus forte teneur en air extérieur en vue d'une dilution supplémentaire des charges.

4. Dispositif selon l'une quelconque des revendications 1 à 3, **caractérisé en ce que** le régulateur (7) peut être réglé en mode manuel.

5. Dispositif selon l'une quelconque des revendications 1 à 4, **caractérisé en ce que** des capteurs de grandeurs perturbatrices supplémentaires suivantes peuvent être utilisés comme signaux d'entrée pour le régulateur (7) : la température, l'humidité absolue ou relative, la densité, la pression d'air, le flux volumétrique, le flux massique, la composition du gaz, la nature des gaz, la quantité de gaz, le degré d'ionisation de l'air.

6. Dispositif selon l'une quelconque des revendications 1 à 5, **caractérisé en ce que** les grandeurs perturbatrices peuvent être représentées visuellement sur un écran, ou acoustiquement, par exemple, par un signal sonore, lorsque lesdites grandeurs sont supérieures à une valeur de consigne.

7. Dispositif selon l'une quelconque des revendications 1 à 6, **caractérisé en ce que** les grandeurs perturbatrices peuvent être éditées en externe par l'intermédiaire d'une ou de plusieurs interfaces, telles que des interfaces de données sérielles, des interfaces de données parallèles, des interfaces USB ou un modem Internet, un modem sans fil/UMTS ou LON.

8. Dispositif selon l'une quelconque des revendications 1 à 7, **caractérisé en ce que** les grandeurs perturbatrices peuvent être stockées pendant une durée déterminée dans le régulateur (7) et, si nécessaire, peuvent être transmises dans une mémoire de masse ou peuvent être éditées sous forme de sortie imprimante.

9. Dispositif selon l'une quelconque des revendications 1 à 8, **caractérisé en ce que** les capteurs sont constitués par des pièces micromécaniques, des systèmes de la nanotechnologie, des capteurs optiques, optoélectroniques ou chimiques, des biocapteurs, ou des combinaisons de ceux-ci.
